# DEMANDE DE BREVET EUROPEEN

(11) **EP 0 802 180 A1**
(43) Date de publication de la demande: **22.10.1997**
(21) Numéro de dépôt: 96400842.9
(22) Date de dépôt: 19.04.1996
(51) Int. Cl.: C07C 403/16, C07C 403/20

(54) **Bèta-méthylène aldéhydes utiles notamment pour la préparation de composés d'intérêt tels que les rétinoides ou les caroténoides et préparation de composés d'intérêt au moyen desdits bèta-méthylène aldéhydes**

(71) Demandeur: CENTRE NATIONAL DE LA RECHERCHE SCIENTIFIQUE (CNRS), 75794 Paris Cédex 16 (FR)
(72) Inventeur: Giraud, Michel, 91150 Etampes (FR); Valla, Alain, 93700 Drancy (FR); Andriamialisoa, Zo, 91940 Les Ulis (FR); Potier, Pierre, 75007 Paris (FR)
(74) Mandataire: Ahner, Francis

(57) **Abrégé**

L'invention concerne les β-méthylène aldéhydes de formule: 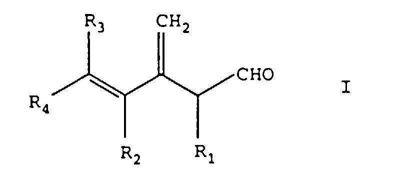 dans laquelle:
R₁, R₂, identiques ou différents sont choisis parmi l'atome d'hydrogène, les radicaux hydrocarbonés carboacycliques, carbocycliques ou hétérocycliques, éventuellement substitués,
R₃, R₄, identiques ou différents sont choisis parmi l'atome d'hydrogène, les radicaux hydrocarbonés carbocycliques, carboacycliques ou hétérocycliques éventuellement substitués, R₃ et R₄ ne pouvant être simultamément l'atome d'hydrogène.

Elle concerne également l'utilisation de ces composés pour la préparation de composés d'intérêt, notamment de la famille des rétinoïdes ou caroténoïdes et leurs analogues structuraux respectifs.

## Description

L'invention a pour objet de nouveaux β-méthylène aldéhydes utiles dans la préparation de composés d'intérêt notamment les dérivés de rétinoïdes ou caroténoïdes et leurs analogues respectifs.

Elle a également pour objet l'utilisation des β-méthylène aldéhydes comme intermédiaires de synthèse desdits composés d'intérêt et un procédé de préparation desdits composés d'intérêt au moyen des β-méthylène aldéhydes précités.

Elle a enfin pour objet un procédé de préparation des β-méthylène aldéhydes et des composés nouveaux utiles notamment dans ledit procédé de préparation.

De nombreuses études ont été déjà consacrées aux synthèses et aux propriétés biologiques de la vitamine A, du rétinal, de l'acide rétinoïque, des rétinoïdes, des caroténoïdes et également aux analogues structuraux respectifs de ces molécules biologiques.

La synthèse de ces molécules biologiques requiert que les réactions soient effectuées selon une stéréospécificité déterminée étant donné les structures E ou Z des doubles liaisons éthyléniques présentes le long desdites molécules.

On rappellera ci-après la structure de quatre acides rétinoïques bien connus:

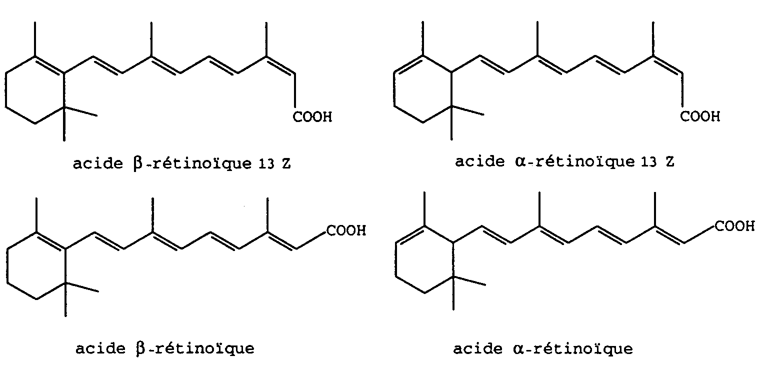

Tous ces dérivés peuvent être utilisés dans des domaines aussi variés que la thérapeutique humaine, la cosmétologie ou l'agroalimentaire.

Dans le cas de la thérapeutique humaine, ces dérivés sont utiles dans le traitement du psoriasis et des acnés sévères et il semblerait de plus que certains rétinoïdes pourraient être utiles dans le domaine de la lutte contre le cancer.

Une des principales méthodes de synthèse pour accéder à ces divers composés est décrite dans le brevet français 1 243 824 et consiste à utiliser comme produit de départ des acétaldéhydes substitués de configuration E, tels que les 9 E α et β-ionylidèneacétaldéhydes dont la structure générale est représentée dans la formule ci-dessous dans laquelle R₄ est un groupe 2, 6, 6-triméthyl-cyclohexène.

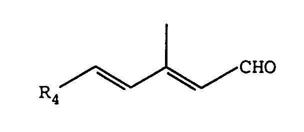

La condensation de ces composés 9 E α et β-ionylidèneacétaldéhydes avec un composé à hydrogène activé comme par exemple un isopropylidène malonate d'alkyle suivie d'une décarboxylation partielle conduit notamment aux acides rétinoïques tels que mentionnés ci-dessus.

Néammoins l'utilisation de dérivés 9 E α et β-ionylidèneacétaldéhydes présente certaines difficultés du fait que ces composés possèdent une configuration 9 E imposée et en conséquence le rendement du procédé de préparation est relativement faible puisqu'il nécessite une étape de purification afin d'éliminer les 9 Z α et β-ionylidèneacétaldéhydes.

Outre cette étape de purification, les voies de synthèse de ces composés font appel à des réactions et à des réactifs dangereux et difficiles à mettre en oeuvre à grande échelle.

Il est donc souhaitable de trouver d'autres intermédiaires de synthèse permettant de pallier ces inconvénients.

Les auteurs de la présente invention ont trouvé de nouveaux composés dont la condensation avec des composés à hydrogène activé conduisait aux mêmes produits que ceux obtenus à partir des 9 E α et β-ionylidèneacétaldéhydes, c'est-à-dire avec la même stéréospécificité.

Par ailleurs, ces composés ne possèdent aucune configuration imposée de type 9 E ou 9 Z et peuvent en conséquence être obtenus avec d'excellents rendements, à grande échelle, pour des coûts très raisonnables.

De plus, ces composés ouvrent la voie à un procédé de préparation de composés d'intérêt avec d'excellents rendements et dans des conditions douces en évitant notamment l'utilisation de bases fortes telles que l'amidure de sodium habituellement employées dans les condensations avec des composés à hydrogène activé.

Les α et β-méthylène aldéhydes selon l'invention répondent à la formule: 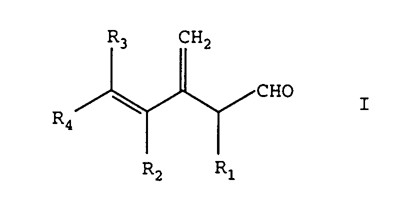 dans laquelle:
R₁, R₂ identiques ou différents sont choisis parmi l'atome d'hydrogène, les radicaux hydrocarbonés carboacycliques, carbocycliques ou hétérocycliques, éventuellement substitués.
R₃, R₄ identiques ou différents sont choisis parmi l'atome d'hydrogène, les radicaux hydrocarbonés carbocycliques, carboacycliques ou hétérocycliques éventuellement substitués, R₃ et R₄ ne pouvant être simultanément l'atome d'hydrogène.

Un groupe carboacyclique est une chaîne carbonée linéaire ou ramifiée comportant éventuellement une ou plusieurs insaturations éthyléniques ou acétyléniques. Les radicaux carboacycliques peuvent être éventuellement substitués. Les groupes carboacycliques comportent généralement entre 1 et 12 atomes de carbone et 0 à 5 insaturations éthyléniques ou acétyléniques.

De façon générale, un groupe carbocyclique ou hétérocyclique est un système monocyclique aromatique ou non aromatique saturé ou insaturé, un système polycyclique aromatique ou non aromatique saturé ou insaturé, ou un système ponté saturé ou insaturé. Ces groupes peuvent être éventuellement substitués.

Les hétéroatomes faisant partie des hétérocycles sont choisis parmi les atomes d'azote, d'oxygène, de phosphore ou de soufre.

Les radicaux R₁ à R₄ peuvent présenter un ou plusieurs centres d'asymétrie et rendre ainsi les composés de formule (I) optiquement actifs.

Les substituants (Z) admissibles sont tous des substituants qui n'interfèreront pas avec la réaction mettant en jeu la fonction aldéhyde, notamment la réaction de cette fonction aldéhyde avec l'hydrogène activé d'un réactant.

Le nombre de substituants (Z) ne devra pas être tel que ces substituants constituent un encombrement stérique trop important susceptible de rendre la molécule instable ou d'accès trop difficile.

Dans le cas des groupes carbocycliques, carboacycliques ou hétérocycliques, les substituants (Z) admissibles sont identiques ou différents et sont choisis parmi les atomes d'halogène (I, Cl, Br, F), les radicaux alkyles, haloalkyles, hydroxy, alcoxy, haloalcoxy, thiol, alkylthio, haloalkylthio, alkylsulfinyle, haloalkylsulfinyle, alkylsulfonyle, haloalkylsulfonyle, alcoxycarbonyle, haloalcoxycarbonyle, alkylcarbonyloxy, haloalkylcarbonyloxy, alkylcarbonyle, haloalkylcarbonyle, amino, alkylamino, haloalkylamino, dialkylamino, dihaloalkylamino, alkylhaloalkylamino, nitro, cyano, oxo, acétal éventuellement cyclique, imine, oxime, carboxyamido, un reste -S(O)ₘ-R dans lequel R est un groupe amino, alkylamino, dialkylamino et m = 0, 1, 2.

Le préfixe alkyle recouvre principalement des restes alkyles linéaires ou ramifiés de 1 à 12 atomes de carbone, avantageusement de 1 à 6.

Le préfixe halo signifie mono ou polyhalogéné. Le nombre de substituants (Z) est généralement compris entre 1 et 6.

Le nombre de substituants (Z) est généralement compris entre 1 et 6.

De préférence, les systèmes monocycliques peuvent être représentés par la formule: 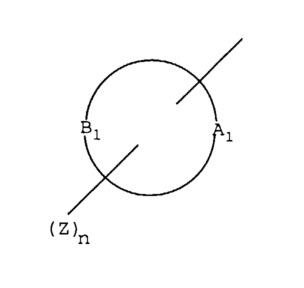 dans laquelle B₁ représente un carbone saturé ou insaturé et A₁ représente une chaîne d'atomes qui ensemble avec B₁ forme un système monocyclique contenant de 0 à 3 double liaisons. A₁ peut comprendre 2 à 7 atomes de carbone ou peut comprendre une combinaison de 2 à 6 atomes de carbone et 1 à 3 hétéroatomes qui peuvent être choisis indépendamment parmi N, O, S, P ou un autre hétéroatome.

Les systèmes comprenant des hétéroatomes peuvent dans certains cas porter des atomes d'oxygène comme dans les systèmes aromatiques contenant un groupe N-oxyde ou contenant un groupe sulfinyle, sulfonyle, sélénoxyde et phosphine oxyde.

Certains carbones des cycles formés par A₁ et B₁ peuvent porter des groupements carbonyle, thiocarbonyle, méthylidène, oxime ou imino éventuellement substitués par un groupe C₁-C₆ alkyle, C₃-C₈ cycloalkyle, C₆-C₁₀ aryle lesdits groupes étant éventuellement substitués par 1 à 6 groupements (Z) tels que définis précédemment.

Le groupe désigné par (Z) représente un ou plusieurs substituants choisis indépendamment parmi le groupe de substituants définis précédemment pour (Z). En général, n = 0 à 6.

Parmi les systèmes bicycliques, on peut citer les systèmes aromatiques comme le naphtalène, le benzimidazole, le biphényle, l'indène, l'azulène...

Parmi les systèmes tricycliques, on peut citer les systèmes aromatiques comme l'anthracène, le phénanthrène, le fluorène, l'indacène, l'acénaphtène...

Parmi les systèmes pontés, on peut citer le norbornane, le pinène, le camphène, le bicyclo-[3, 2, 1]-octane...

Selon une variante préférée, les radicaux R₃, R₄ sont choisis parmi les radicaux hydrocarbonés carbocycliques ou hétérocycliques.

Selon une variante préférée, les radicaux R₃ ou R₄ sont un radical hydrocarboné et l'autre radical est un atome d'hydrogène.

Selon une autre variante préferée prise en combinaison avec la précédente, R₄ est un radical hydrocarboné.

De préférence, R₄ est un cycle à 6 atomes de carbone, aromatique, mono ou di-insaturé substitué éventuellement.

De préférence, les substituants sont choisis pami les radicaux C₁-C₄ alkyle, hydroxy, C₁-C₄ alcoxy, oxo.

De préférence encore, R₂ est un atome d'hydrogène.

Selon une autre variante préférentielle, R₁ est un atome d'hydrogène.

Selon une variante préférée, le motif: 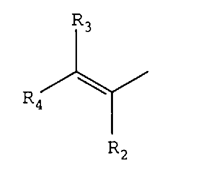 est un reste terminal d'un composé choisi parmi les molécules biologiques telles que les rétinoïdes, les caroténoïdes et leurs analogues structuraux respectifs.

Parmi ces composés, on peut citer les dérivés dont les formules suivent:

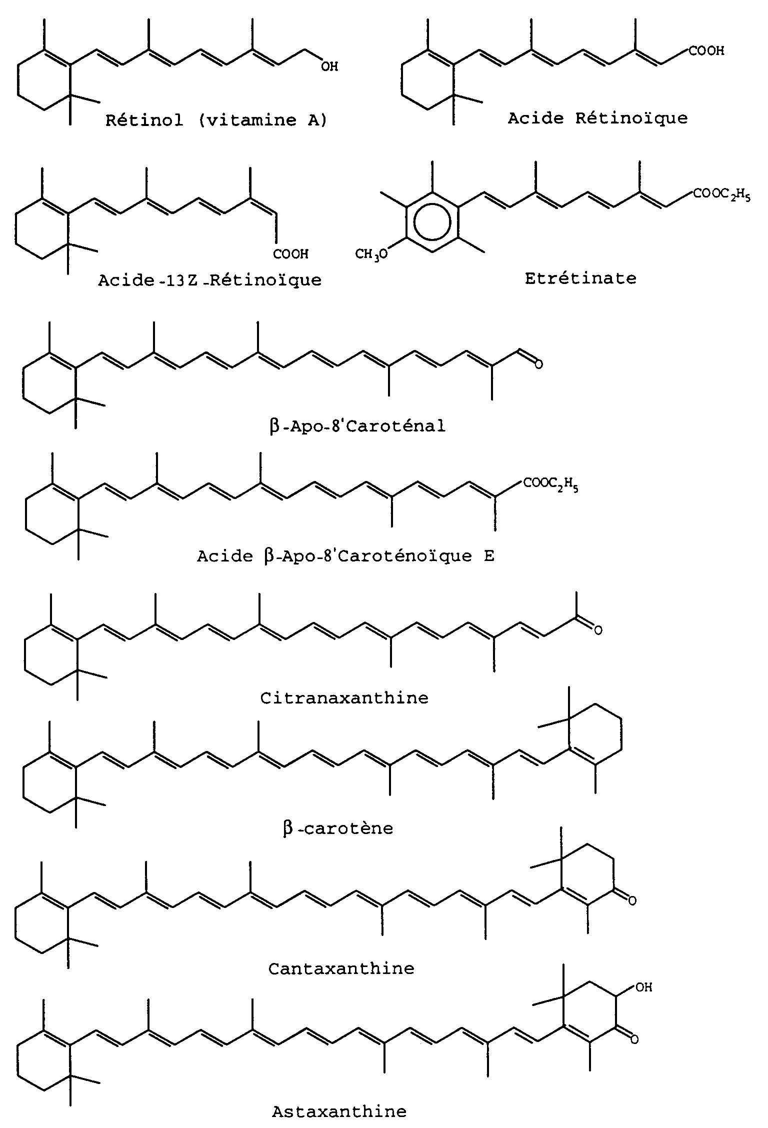

De préférence, R₄ est un radical cyclohexényle substitué en position 2, 6, 6 par des radicaux méthyles et répond aux formules:

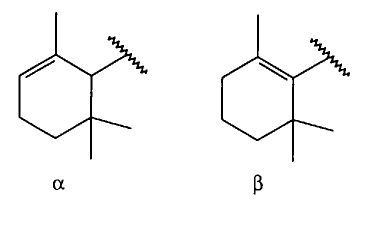

L'invention concerne également un procédé de préparation de composés d'intérêt caractérisé en ce que l'on fait agir un composé de formule (I) tel que défini ci-dessus, avec un composé présentant un hydrogène susceptible d'être activé, de formule (II) selon la réaction suivante: 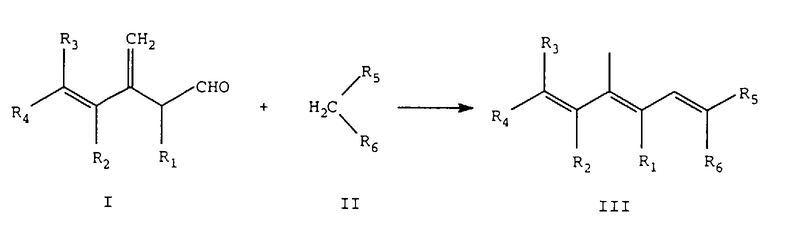 R₁, R₂, R₃, R₄, ont l'une des significations indiquées précédemment et R₅, R₆ identiques ou différents sont un atome d'hydrogène, un radical alkyle inférieur ou un groupe électroattracteur à condition que l'un des radicaux soit un groupe électroattracteur,
ou R₅ est un atome d'hydrogène et R₆ est un groupe -CR₇ = C(R₈)₂ où R₇ est un atome d'hydrogène ou un radical alkyle inférieur, un radical alkyle sulfonyle ou aryle sulfonyle et R₈ est un groupe électroattracteur.

Les groupes électroattracteurs sont bien connus et sont notamment choisis parmi les groupes esters, cyano, carboxylique, alkyle sulfonyle ou aryle sulfonyle.

Par l'expression "hydrogène activé", on entend de façon générale un atome d'hydrogène situé sur un méthylène activé ou un méthyle activé par vinylogie.

La réaction est effectuée en présence d'un agent activant ledit hydrogène. La réaction peut être effectuée en milieu basique, notamment en présence d'un amidure alcalin. Toutefois, et c'est un des avantages supplémentaires de la présente invention, la réaction peut être effectuée dans des conditions douces en présence d'un hydroxyde d'ammonium quaternaire tel que le Triton B ou d'une base équivalente.

On récupère un composé de formule III, que l'on transforme éventuellement. 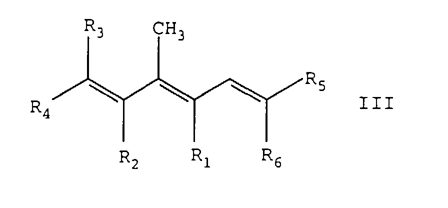 De préférence, comme cela a déjà été mentionné auparavant, le composé de formule II participe au squelette d'un composé choisi parmi les rétinoïdes, les caroténoïdes et leurs analogues structuraux respectifs tels qu'ils sont par exemple présentés dans les formules de ces composés mentionnés précédemment.
De préférence, le composé de formule II répond à la formule 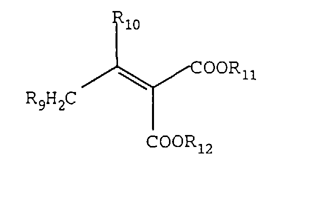 dans laquelle:
R₉ est un atome d'hydrogène ou un radical alkyle éventuellement substitué notamment méthyle,
R₁₀ est un atome d'hydrogène ou un radical alkyle,
R₁₁, R₁₂ sont tels que les fonctions esters COOR₁₁ ou COOR₁₂ soient hydrolysables en fonctions acides et le composé de formule III répond à la formule : 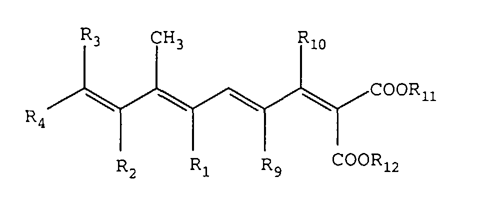 Selon une variante préférée, le rapport molaire I/II est compris entre 0,2 et 2.
De préférence encore, le composé de formule II est un diester de l'acide isopropylidène malonique. Parmi les diesters on peut citer ceux en C₁-C₄, comme l'ester méthylique ou éthylique.
Le composé de formule III est selon une variante, hydrolysé en composé de formule IV 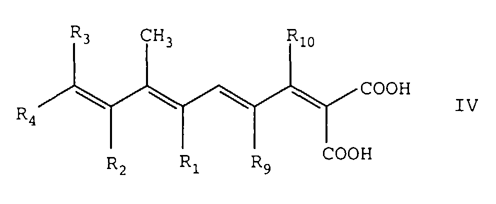 celui-ci étant ensuite décarboxylé pour obtenir le composé de formule V 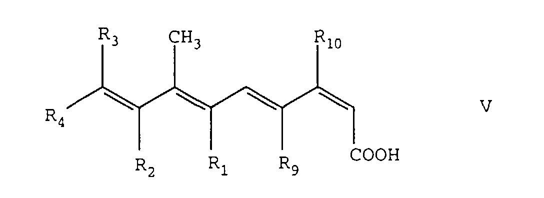 ou VI:

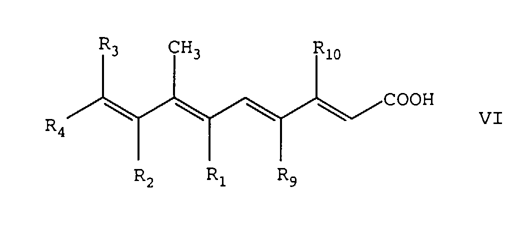

La réaction d'hydrolyse et de décarboxylation est effectuée de manière connue.

La transformation des acides de formule V et VI en alcool ou aldéhyde correspondant est réalisée de manière connue.

L'invention a également pour objet un procédé de préparation de composés de formule I.

Le procédé est caractérisé en ce qu'on fait réagir une cétone éthylénique de formule: 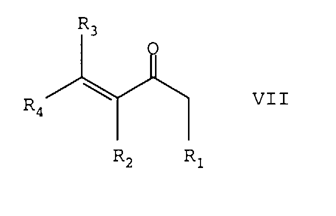 avec un formiate de formule HCOOR₁₃
R₁₃ étant notamment un reste alkyle linéaire ou ramifié, pour obtenir le composé de formule: 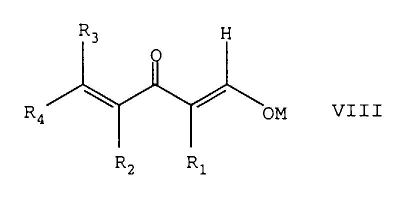 M est un métal alcalin ou un atome d'hydrogène.

Cette réaction est généralement effectuée en présence d'une base telle que les alcoolates alcalins, les hydrures alcalins, les amidures alcalins etc., dans un solvant aprotique polaire ou apolaire tel que les hydrocarbures saturés, cycliques ou acycliques comme l'hexane, le cyclohexane, les hydrocarbures aromatiques comme le toluène, le xylène, les éthers comme l'éther éthylique, le tétrahydrofuranne...
Le composé de formule VIII est ensuite acétalisé en composé de formule: 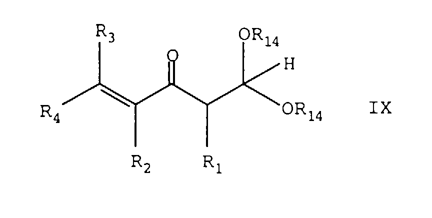 les radicaux R₁₄ étant notamment des radicaux alkyle linéaires ou ramifiés ou représentent un cycle comme (CH₂)ₙ. avec n = 1, 2, 3, 4. La réaction d'acétalisation est effectuée de manière connue.

Par méthylénation de la fonction cétone, notamment à l'aide de la réaction de Wittig, on fait réagir un halogénure de méthyle triphénylphosphonium ou de méthyle trialkyl-phosphonium pour obtenir le β-méthylène acétal de formule X

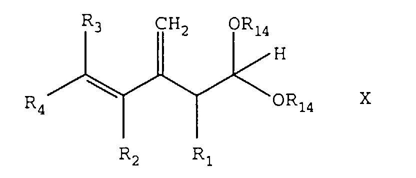

Le β-méthylène acétal X est ensuite hydrolysé pour donner le β-méthylène aldéhyde de formule I.

L'invention a également pour objet les nouveaux composés de formules VIII, IX et X utiles notamment dans la préparation des composés de formule I.

L'invention est maintenant illustrée par les exemples suivants donnés à titre indicatif.

### Exemple 1 :Préparation du composé de formule

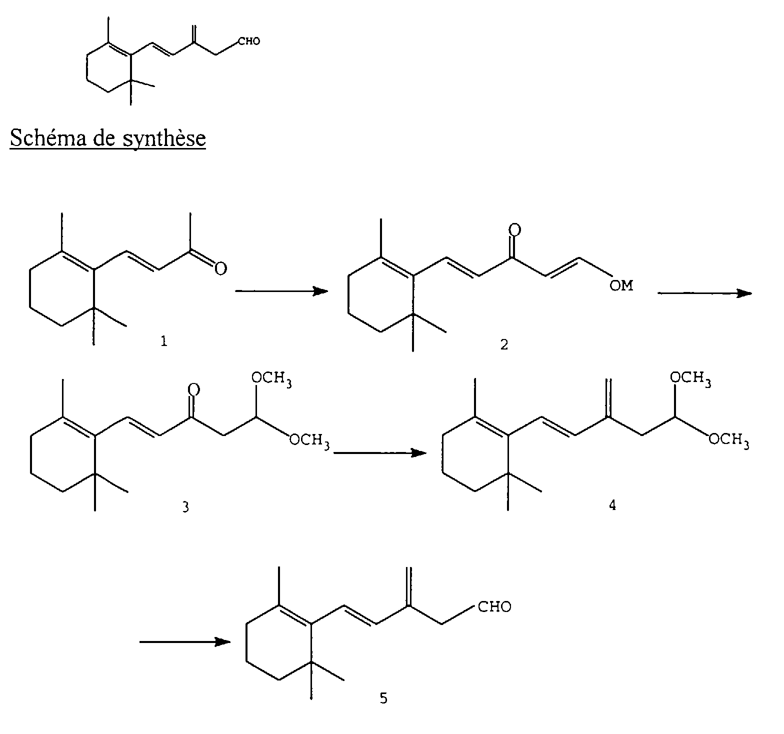

### a) Préparation du composé n° 2 (sel de sodium)

Ajouter vers 10°C, en 15 mn un mélange de 192 g (1 mole) de β-ionone et de 120 g (2 moles) de formiate de méthyle (employé en excès à cause de sa volatilité) à une suspension de 64,8g (1,2 moles) de méthylate de sodium dans 200 ml de cyclohexane anhydre . On laisse revenir à la température ambiante (environ 30mn), on filtre et lave le sel de sodium au cyclohexane. Poudre beige, presque blanche. Rdt ≥ 90%.

Le composé n° 2 où M = H est préparé par acidification du sel de sodium à 0°C par une solution d'HCI à 10 %. Le produit est rapidement extrait à l'éther, lavé à l'eau, séché, conservé à froid.

### b) 1 - Préparation du composé n° 3

On ajoute goutte à goutte vers 10°C, 10 ml de chlorure d'acétyle à 30 g de sel de sodium en suspension dans 100 ml de méthanol anhydre et 30 ml de formiate de méthyle. Après 30 mn d'agitation, on neutralise lentement par du bicarbonate de sodium. On filtre et lave le précipité au cyclohexane. On distille le méthanol et le cyclohexane sous pression réduite (Rdt ≥ 85%). Le produit ne nécessite pas de purification pour l'étape suivante (Echantillon analytiquement pur obtenu par chromatographie sur colonne de silice (pentane-éther, 60v-40v).

### b) 2-Préparation du composé n° 3

Selon le même principe que b) 1, mais avec de l'isopropanol anhydre on obtient le composé attendu (Rdt ≥ 75%).

### c) Préparation du composé n° 4

Sous argon, on ajoute en 10 mn à la spatule 7,2 g (20 mmol) de CH₃Pφ₃ (bromure de méthyle triphénylphosphonium) à 15 ml de *n*-*butyl* lithium (1,6M, 30 mmol dans hexanes) dans 30 ml d'éther anhydre. Après 4 h d'agitation à température ambiante, on refroidit à 0°C et on ajoute lentement (15 mn) 11,5 g de β-céto-acétal diméthylique en solution dans 30 ml d'éther anhydre. Après 2 h d'agitation à température ambiante, on refroidit vers 0°C et on hydrolyse par une solution de chlorure d'ammonium. On filtre et lave les résidus gommeux au pentane. Les phases organiques sont réunies, filtrées et l'huile obtenue après distillation des solvants sous pression réduite est purifiée par chromatographie sur colonne de silice (pentane-éther, 90v-10v). Rdt 40%

Il est possible d'améliorer cette synthèse de la façon suivante:

On ajoute 35,7g (0,1 mole) de CH₃Pφ₃, en 15 mn à 11,2 g (0,1 mole) de *tert*-BuOK dans 100 ml de cyclohexane anhydre. On chauffe à 45°C pendant 15 mn puis on refroidit vers 10°C. On ajoute alors, lentement 26,6g (0,1 mole) de β-cétoacétal dans 50 ml de cyclohexane anhydre. Après 15 mn à température ambiante, on distille le solvant sous pression réduite, le β-méthylène-acétal diméthylique est adsorbé sur 150g desilice et élué par 1l de chlorure de méthylène. Rdt ≥ 80%.

### d) Préparation du composé n° 5

10g d'acétal dans 40 ml de cyclohexane et 15 ml d'acide formique sont agités environ 4h à température ambiante. On ajoute 40g de glace, on décante et lave la phase aqueuse au cyclohexane. Les phases organiques sont réunies et lavées avec une solution de bicarbonate de sodium, puis à l'eau jusqu'à neutralité des eaux de lavages. Huile jaune, très pâle. Rdt ≥ 95%.

Le rendement global de la réaction (passage du composé 1 au composé 5) est ≥ à 72,5%.
On donne ci-après les caractéristiques des composés 2, 3, 4, et 5 obtenus.
composé n° 2 (sel de sodium)
IR (Kbr) 2940, 2865, 1644, 1597, 1500, 1450, 1345, 1170, 980, 770, 565.
¹H RMN (D₂O): 0,88 (s, 6H, C₆-CH₃); 1,35 m, 2H, C₅-H); 1,65 (m, 2H, C₄-H); 1,77 (s, 3H, C₂-CH₃); 1,87 (m, 2H, C₃-H); 5,20 (d, 1H, *J*=12, C₁₀-H); 6,10 (m, 1H, C₈-H); 6,85 (d, 1H, *J*=16, C₇-H); 8,95 (d, 1H, *J*=12, C₁₁-H).
composé n° 2
IR 2960, 2930, 2870, 1610, 1470, 1300, 980, 770, 565.
¹H RMN (CDCl₃): 1,00 (s, 6H, C₆-CH₃); 1,49 (m, 2H, C₅-H); 1,58 (m, 2H, C₄-H); 1,71 (s, 3H, C₂-CH₃); 2;00 (m, 2H, C₃-H); 5,51 (d, 1H, *J*=3,5, C₁₀-H); 5,86 (d, 1H, *J*=16, C₇-H); 7,33 (d, 1H, *J*=16, C₈-H); 8,44 (d, 1H, *J*=3,5, C₁₁-H)
composé n° 3
IR (Film ν cm⁻¹) 2935, 2875, 2830, 1697, 1664, 1610, 1465, 1370, 1320, 1130, 1080, 980.
¹H RMN (CDCl₃): 1,09 (s, 6H, C₆-CH₃); 1,40 (m, 2H, C₅-H); 1,75 (m, 2H, C₄-H); 1,79(s, 3H, C₂-CH₃); 2,08 (m, 2H, C₃-H); 2,92 (d, 2H, *J*=5,5, C₁₀-H); 3,41 (s, 6H, OCH₃); 4,38 (t, 1H, *J*=5,5 C₁₁-H); 6,18 (d, 1H, *J*=16, C₇-H); 7,28 (d, 1H, *J*=16, C₆-H).
¹³C RMN (CDCl₃): 18,7 (C₄); 21,8 (C₆-CH₃); 28,8 (C₂-CH₃); 33,6 (C₃); 33,9 (C₆); 39,8 (C₅); 44,2 (C₁₀); 54,0 (OCH₃); 102,3 (C₁₁); 130,9 (C₇); 136,1 (C₂); 136,6 (C₁); 143,2 (C₈); 196,5 (C₉).
analogue du composé n° 3 (acétal isopropylique)
IR (film): 2970, 2932, 1692, 1665, 1607.
¹H RMN (CDCl₃): 1,04 (s, 6H, C₆-CH₃); 1,1 et 1,16 (2d, 12H, *J*=7, OCH(CH₃)₂; 1,46 (m, 2H, C₅-H); 1,60 (m, 2H, C₄-H); 1,74 (s, 3H, C₂-CH₃); 2,04 (m, 2H, C₃-H); 2,84 (d, 2H, *J*=5,5, C₁₀-H); 3,84 (m, 1H, C₁₂-H); 5,0 (t, 1H, *J*=7, C-₁₁-H); 6,12 (d, *J*=16, C₈-H); 7,28 (d, 1H, C₇-H).
composé n° 4
IR (film): 2930, 1602, 1122, 1068, 971, 888.
¹H RMN (CDCl₃): 6,13 et 5,99 (2d, 2H, *J*=16, C₇-H+C₈-H); 5,02 et 4,99 (2sl, 2H, C₉-H); 4,56 (t, 1H, *J*=5,5, C₁₁-H); 3,32 (s, 6H, OCH₃); 2,55 (d, 2H, *J*=5,5, C₁₀-H); 1,96 (m, 2H, C₄-H); 1,65 (s, 3H, C₂-CH₃); 1,59 (m, 2H, C₅-H); 1,42 m, 2H, C₄-H); 0,97 (s, 6H, C₆-CH₃).
¹³C RMN (CDCl₃): 141,7 (C₁); 137,6 (C₂); 135,1 (C₇); 129,2 (C₉); 127,6 (C₈); 116,5 (CH₂-C₉); 103,7 (C₁₁); 53,3 (OCH₃); 39,5 (C₁₀); 36,1 (C₅); 34,3 (C₆); 32,9 (C₃); 29,0 (C₂-CH₃); 21,7 (C₆-CH₃); 19,4 (C-4).
composé n° 5
IR (film): 2930, 2715, 1727, 1603.
¹H RMN (CDCl₃): 9,60 (t, 1H, *J*=2,7, CHO); 6,12 et 6,02 (2d, 2H, *J*=16,4, C₇-H+C₈-H); 5,20 et 5,07 (2sl, 2H, C₉-H); 3,37 (d, 2H, *J*=2,7 C₁₀-H); 1,97 (m, 2H, C₃-H); 1,64 (s, 3H, C₂-CH₃); 1,56 (m, 2H, C₄-H); 1,44 m, 2H, C₅-H); 0,96 (s, 6H, C₆-CH₃).
¹³C RMN (CDCl₃): 199,5 (C₁₁); 137,6 (C-1); 136,8 (C₂); 133,9 et 129,1 (C₇+C₈); 129,3 (C₉); 118,4 (CH₂-C₉); 44,3 (C₁₀); 39,1 (C₅); 33,8 (C₆); 32,5 (C₃); 28,5 (C₂-CH₃); 21,2 (C₆-CH₃); 18,9 (C₄).

### Exemple 2

### Préparation du composé de formule

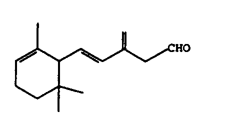

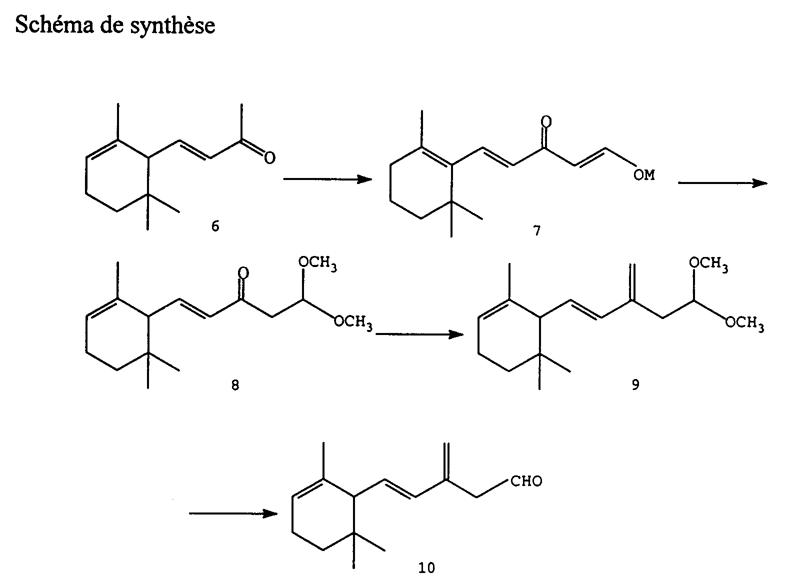

Les conditions opératoires sont identiques à celles de l'exemple 1. On donne ci-après les caractéristiques des composés 7, 8, 9 et 10 obtenus.
composé n°7 (sel de sodium)
IR (Kbr) 2868, 2920, 2868, 1657, 1600, 1500, 1450, 1360, 1220, 990, 770, 545.
composé n° 7
IR 2967, 2921, 2854, 1624, 1585, 1446, 1295, 1216, 986, 775, 551.
¹H RMN (CDCl₃): 0,86 et 0,78 (2s, 6H, C₆-CH₃); 1,3 (m, 2H, C₅-H); 1,57 (s, 3H, C₂-CH₃); 1,96 (m, 2H, C₄-H); 2,23 (d, 1H, J=5, C₁-H); 5,42 (m, 1H, C₃-H); 5,50 (d, 1H, *J*=3,5, C₁₀-H); 5,82 (d, 1H, *J*=16, C₈-H); 6,62 (dd, 1H, *J*=16, *J*=5, C₇-H); 8,41 (d, 1H, *J*=3,5, C₁₁-H).
composé n° 8
IR (Film ν cm⁻¹) 2970, 2920, 2875, 1670, 1625, 1450, 1375, 1130, 1088, 992.
¹H RMN (CDCl₃): 0,89 et 0,81 (2s, 6H, C₆-CH₃); 1,30 (m, 2H, C₅-H); 1,50 (s, 3H, C₂-CH₃); 2,00 (m, 2H, C₄-H); 2,28 (d, 1H, *J*=9, C₁-H); 2,80 (d, 2H, *J*=5, C₁₀-H); 3,30 (s, 6H, OCH₃); 4,77 (t, 1H, *J*=5 C₁₁-H); 5,40 (m, 1H, C₃-H); 6,00 (d, 1H, *J*=16, C₈-H); 6,62 (dd, 1H, *J*=16, *J'*=9, C₇-H).
¹³C RMN (CDCl₃): 207,1 (C₉); 183,9 (C₁); 149,3 (C₇); 131,8 (C₈); 122,7 (C₃); 102,1 (C₁₁); 54,8 (C₁); 54,1 (OCH₃); 43,9 (C₁₀); 32,5 (C₆); 31,2 (C₅); 27,7 (C₂-CH₃); 26,6 (C₆-CH₃); 23,0 (C₄); 19,7 (C₆-CH_{3'}).
composé n° 9
IR (film): 2930, 1602, 1122, 1068, 971, 888.
¹H RMN (CDCl₃): 6,02 (d, 1H, *J*=16, C₈-H); 5,51 (dd, 1H, *J*=16, *J'*=9,5, C₇-H); 5,39 (m, 1H, C₃-H); 4,98 (2s, 2H, C₉-CH₂); 4,50 (t, 1H, *J*=5,5, C₁₁); 3,30 (s, 6H, OCH₃); 2,49 (d, 2H, *J*=5,5, C₁₀-H); 2,12 (d, 1H, *J*=9,5, C₁-H); 1,98 (m, 2H, C₄-H); 1,55 (s, 3H, C₂-CH₃); 1,26 (m, 2H, C₅-H); 0,85 et 0, 83 (2s, 6H, C₆-CH₃).
¹³C RMN (CDCl₃): 140,8 (C₂); 132,8 (C₇); 131,8 (C₈); 128,0 (C₉); 120,3 (C₃); 115,5 (CH₂-C₉); 103,1 (C₁₁); 54,3 (OCH₃); 52,6(C₁); 35,8 (C₁₀); 32,0 (C₆); 31,4 (C₅); 26,6 (C₂-CH₃); 22,6 (C₄); 19,2 (C₆-CH₃).
composé n° 10
IR (film): 2930, 2715, 1727, 1603.
¹H RMN (CDCl₃): 9,60 (t, 1H, *J*=2,7, C₁₁-H); 6,13 (d, 1H, *J*=16, C₈-H); 5,42 (dd, 1H, *J*=16, *J'*=5, C₇-H); 5,35 (sl, 1H, C₃-H); 5,18 et 5,03 (2s, 2H, C₉-CH₂); 3,21 (d, 2H, *J*=2,7 C₁₀-H); 2,14 (d, 1H, J=5, C₁-H); 1,99 (m, 2H, C₄-H); 1,53 (s, 3H, C₂-CH₃); 1,27 (m, 2H, C₅-H); 0,87 et 0,76 (2s, 6H, C₆-CH₃).
¹³C RMN (CDCl₃): 220,3 (C₁₁); 137,6 (C₂); 133,7 (C₇); 133,9 et 132,6 (C₈); 121,7 (C₉); 121,5 (C₃); 118,4 (CH₂-C₉); 54,7 (C₁);48,0 (C₁₀); 32,5 (C₆); 31,8 (C₅); 27,5 (C₂-CH₃); 27,1 (C₆-CH₃); 23,0 (C₄); 22,8 (C₆CH₃)).

### Exemple 3 Préparation du composé n° 12

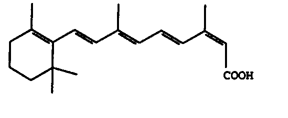

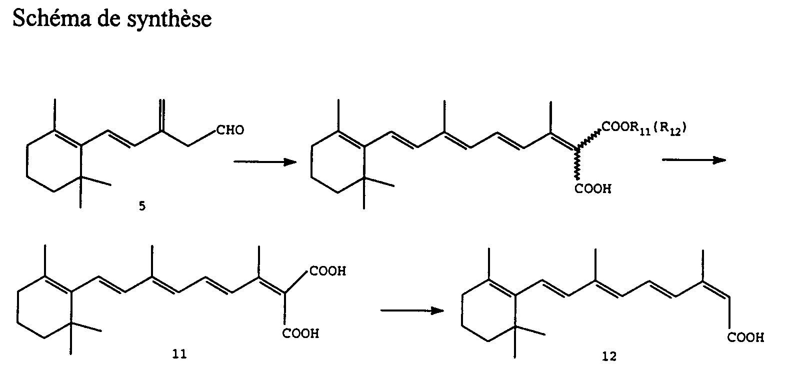

### a) Préparation du composé n° 11

1,3 g d'aldéhyde sont ajoutés lentement sous argon, entre 0 et 5°C, à une solution de 1,5 g d'isopropylidène malonate diméthylique dans 8 ml de triton B (40% dans le méthanol). Après 2 h à température ambiante, on acidifie par une solution glacée d'HCI à 10% et on extrait à l'éther. L'acide ester est extrait par une solution aqueuse de soude à 5%, puis libéré de son sel de sodium par une solution d'HCI à 10%. Le produit brut (Rdt 95%) est saponifié par une solution 0,75N de soude (60% H₂0, 40% MeOH), 1 h 30 à ébullition (Rdt 100%). Cristaux jaunes, F 198°C.

### b) Préparation du composé n° 12

1,5 g de diacide sont chauffés 2 h à ébullition dans 50 ml de diméthyl-2, 6-pyridine. Après distillation de la base sous pression réduite, on refroidit vers 0°C et on acidifie par une solution d'HCI à 10%. L'acide est extrait à l'éther. Rdt: 100%. L'acide cristallise de l'éther. Les caractéristiques des composés 11 et 12 sont les suivantes
composé n° 11 (dérivé de l'aldéhyde 5)
IR (Kbr): 2927, 1676, 1611, 1571, 1455, 1255, 979, 722.
¹H RMN (CDCl₃): 7,17 (m, 1H, C₁₁-H); 6,95 (d, 1H, *J*=16, C₁₂); 6,28 (m, 2H, C₇+C₁₀-H); 6,18 (d, 1H, *J*=16, C₈); 2,13 (s, 3H, C₁₃-CH₃); 2,02 (m, 2H, C₃-H); 1,98 (s, 3H, C₉-CH₃); 1,58 (s, 3H, C₉-CH₃); 1,50 (m, 2H, C₄-H); 1,46 m, 2H, C₅-H); 1,00 (s, 6H, C₆-CH₃).
¹³C RMN (CDCl₃): 167,7 et 167,3 (C-15+C₁₅-COOH); 146,3 (C-14); 143,9 (C-13); 141,1 (C-9); 137,7 (C-1); 137,3 (C-12); 133,5 (C-7); 130,6 (C-8); 130,4 (C-11); 128,8 (C-10); 126,7 (C-2); 34,3 (C-6); 34,1 (C-5); 29,2 (C₂-CH₃); 21,9 (C₆-CH₃); 19,2 (C-4); 16,0 (C₉-CH₃); 13,1 (C₁₃-CH₃).
composé n° 12 (13 *Z* ,dérivé de l'aldéhyde 5)
IR (Kbr): 2960, 2921, 2861,, 1683, 1591, 1387, 1249, 972
¹H RMN (CDCl₃): 7,72 (d, 1H, *J*=16, C₁₂-H); 7,02 (m, 1H, C₁₁-H); 6,27, 6,25, 6,17 (m, 3H, C₇+C₈+C₁₀-H); 5,64 (sl, 1H, C₁₄-H); 2,08, (s, 3H, C₁₃-CH₃); 2,00 (m, 2H, C₄-H); 1,98 (s, 3H, C₉-CH₃); 1,69 (s, 3H, C₂-CH₃); 1,59 (m, 2H, C₅-H); 1,46 (m, 2H, C₃-H); 1,00 (s, 6H, C₆-CH₃).

### Exemple 4

### Préparation du composé n° 14

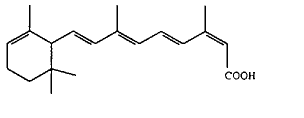

Les étapes a) et b) sont identiques à celles de l'exemple 3. 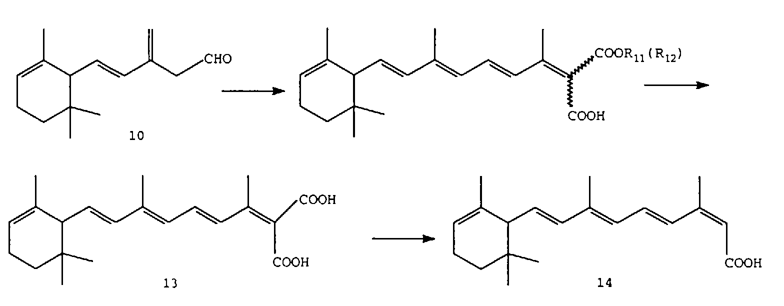 Les caractéristiques des composés 13 et 14 sont les suivantes
composé n° 13 (dérivé de l'aldéhyde 10)
IR (Kbr): 2940, 2919, 2604, 1710, 1575, 1251, 970, 722.
¹H RMN (CDCl₃): 7,05 (m, 2H, C₁₁+C₈-H; 6,12 (d, 1H, *J*=10, C₁₀-H); 6,04 (d, 1H, *J*=16, C₁₂-H); 5,60 (dd, 1H, *J*=16, *J'*=9 C₇-H); 5,34, (m, 1H, C₃-H); 2,18 (s, 3H, C₉-CH₃); 2,10 (d, 1H, *J*=9, C₁-H); 1,90 (m, 2H, C₄-H); 1,87 (s, 3H, C₂-CH₃); 1,50 (s, 3H, C₁₃-CH₃); 1,20 (m, 2H, C₅-H); 0,85 et 0,70 (2s, 6H, C₆-CH₃).
¹³C RMN (CDCl₃): 185,8 (CO); 168;8 (C₁₄); 168,3 (C₁); 143,0 (C₁₃); 134,0 (C₉); 135,7, 133,7, 133,6, 133,0 (C₇+C₈+C₁₀+C₁₁); 129,5 (C₁₂); 121,0 (C₃); 54,8 (C₁); 32,5 (C₆); 31,6 (C₄); 27,7 (C₂-CH₃); 26,9 (C₆-CH₃); 16,2 (C₆CH₃); 13,3 (C₆-CH₃).
composé n° 14 (13 *Z* ,dérivé de l'aldéhyde 10)
IR (Kbr): 2934, 1683, 1604, 1572, 1249, 972
¹H RMN (CDCl₃): 7,67 (d, 1H, *J*=16, C₁₂); 6,80 (m, 1H, C₁₁-H); 5,90 (m, 2H, C₈+C₁₀); 5,70 (m, 1H, C₃-H); 5,40 (dd, 1H, C₇-H); 2,28 (d, 1H, C₁-H); 2,0 (s, 3H, C₂-CH₃); 1,90 (s, 3H, C₉-CH₃); 1,85 (s, 3H, C₁₃-CH₃); 1,50 (m, 2H, C₄-H); 1,30 (m, 2H, C₅-H); 0,9 et 0,8 (2s, 6H, C₆-CH₃).

## Revendications

1. β-méthylène aldéhydes de formule 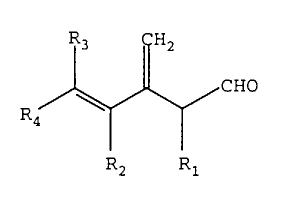 dans laquelle
R₁, R₂, identiques ou différents sont choisis parmi l'atome d'hydrogène, les radicaux hydrocarbonés carboacycliques, carbocycliques ou hétérocycliques, éventuellement substitués,
R₃, R₄ identiques ou différents sont choisis parmi l'atome d'hydrogène, les radicaux hydrocarbonés carbocycliques, carboacycliques ou hétérocycliques éventuellement substitués, R₃ et R₄ ne pouvant être simultanément l'atome d'hydrogène.

2. β-méthylène aldéhydes selon la revendication 1, caractérisés en ce que R₃, R₄, identiques ou différents, sont choisis parmi l'atome d'hydrogène, les radicaux hydrocarbonés carbocycliques ou hétérocycliques, éventuellement substitués, R₃ et R₄ ne pouvant être simultanément l'atome d'hydrogène.

3. β-méthylène aldéhydes selon la revendication 1 ou 2, caractérisés en ce que R₃ ou R₄ est un radical hydrocarboné et l'autre radical est un atome d'hydrogène.

4. β-méthylène aldéhydes selon la revendication 3, caractérisés en ce que R₄ est un radical hydrocarboné éventuellement substitué.

5. β-méthylène aldéhydes selon l'une des revendications 1 à 4, caractérisés en ce que dans le cas des groupes carbocyliques, carboacycliques ou hétérocycliques, les substituants (Z) sont identiques ou différents et sont choisis parmi les atomes d'halogène (I, CI, Br, F), les radicaux alkyle, haloalkyle, hydroxy, alcoxy , haloalcoxy , thiol , alkylthio, haloalkylthio alkylsulfinyle , haloalkylsulfinyle, alkylsulfonyle, haloalkylsulfonyle, alcoxycarbonyle, haloalcoxycarbonyle, alkylcarbonyloxy, haloalkylcarbonyloxy, alkylcarbonyle, haloalkylcarbonyle, amino, alkylamino, haloalkylamino, dialkylamino, dihaloalkylamino, alkylhaloalkylamino, nitro, cyano, oxo, acétal éventuellement cyclique, imine, oxime, carboxyamido un reste -S(O)ₘ,-R dans lequel R est un groupe amino, alkylamino, dialkylamino et m = 0, 1, 2.

6. β-méthylène aldéhydes selon l'une des revendications 1 à 5, caractérisés en ce que lorsque les radicaux hydrocarbonés carboacycliques, carbocycliques ou hétérocycliques sont substitués, le nombre de substituants est compris entre 1 et 6.

7. β-méthylène aldéhydes selon l'une des revendications précédentes, caractérisés en ce que R₄ est un cycle à 6 atomes de carbone, aromatique, saturé, mono ou di-insaturé, éventuellement substitué par des radicaux C₁-C₄ alkyle, hydroxy, C₁-C₄ alcoxy ou une fonction oxo.

8. β-méthylène aldéhydes selon l'une des revendications 1 à 7, caractérisés en ce que R₂ est un atome d'hydrogène.

9. β-méthylène aldéhydes selon l'une des revendications 1 à 8, caractérisés en ce que R₁ est un atome d'hydrogène.

10. β-méthylène aldéhydes selon l'une des revendications 1 à 9, caractérisés en ce que le motif: 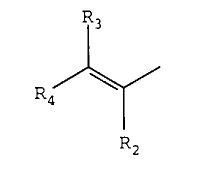 est un reste terminal d'un composé choisi parmi les rétinoïdes, les caroténoïdes et leurs analogues structuraux respectifs.

11. β-méthylène aldéhydes selon la revendication 10, caractérisés en ce que les composés sont choisis parmi les composés suivants:

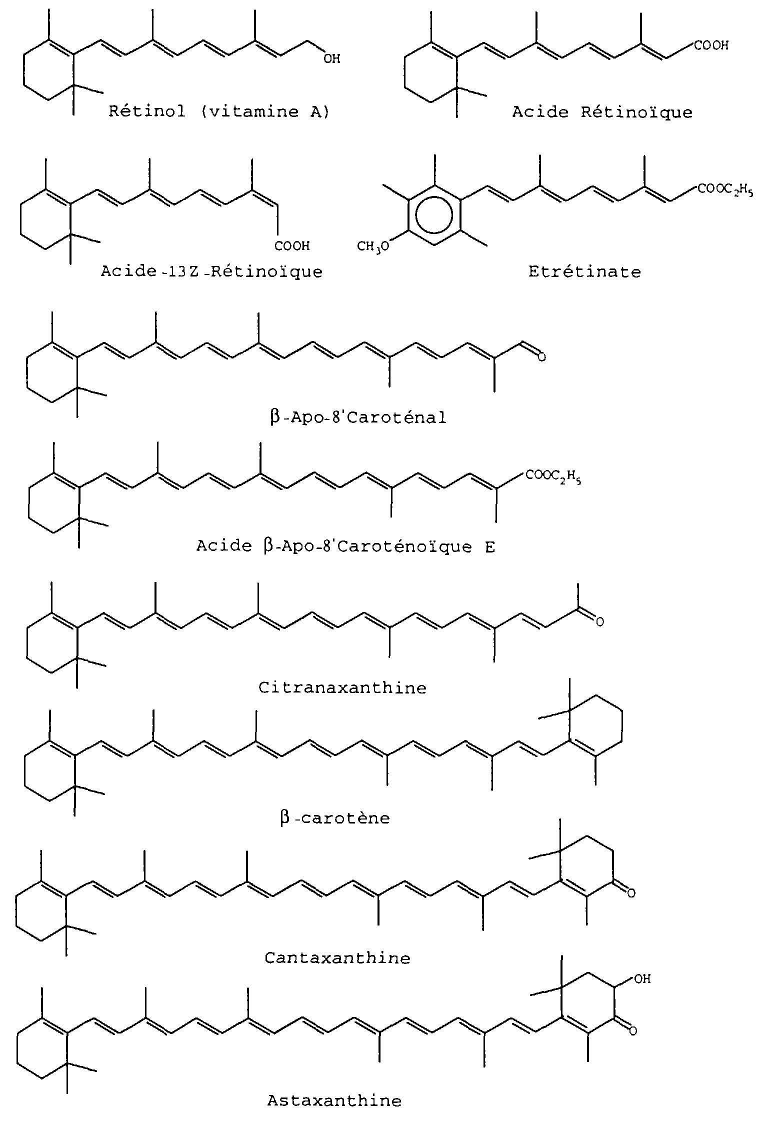

12. β-méthylène aldéhydes selon la revendication 11, caractérisés en ce que R₄ est un radical 2, 6 ,6-triméthylcyclohexényle éventuellement substitué.

13. β-méthylène aldéhydes selon la revendication 12, caractérisés en ce que R₄ répond à la formule:

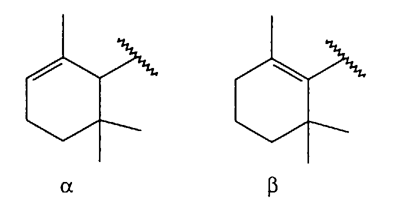

14. Utilisation des composés selon l'une des revendications 1 à 13, comme intermédiaires de synthèse de composés d'intérêt.

15. Procédé de préparation de composés d'intérêt caractérisé en ce qu'on fait réagir un composé de formule (I) selon l'une des revendications 1 à 13, avec un composé présentant un hydrogène susceptible d'être activé, de formule II 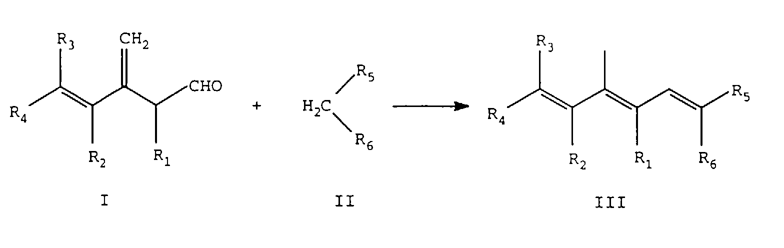 R₁, R₂, R₃, R₄ ayant l'une des significations des revendications 1 à 10,
R₅, R₆ identiques ou différents sont un atome d'hydrogène, un radical alkyle inférieur ou un groupe électroattracteur à condition que l'un des radicaux soit un groupe électroattracteur,
ou R₅ est un atome d'hydrogène et R₆ est un groupe -CR₇ = C(R₈)₂ où R₇ est un atome d'hydrogène ou un radical alkyle inférieur et R₈ est un groupe électroattracteur, en présence d'un agent activant ledit hydrogène puis à récupérer un composé de formule III 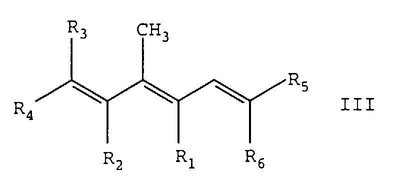 et à transformer éventuellement ledit composé de formule III.

16. Procédé de préparation selon la revendication 15, caractérisé en ce que le composé de formule II participe au squelette d'un composé choisi parmi les rétinoïdes, les caroténoïdes et leurs analogues structuraux respectifs.

17. Procédé de préparation selon la revendication 16, caractérisé en ce que le composé de formule II répond à la formule: 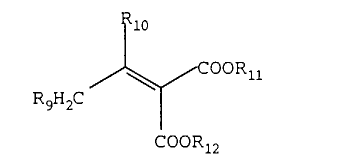 dans laquelle:
R₉ est un atome d'hydrogène ou un radical alkyle éventuellement substitué, notamment méthyle,
R₁₀ est un atome d'hydrogène ou un radical alkyle
R₁₁, R₁₂ sont tels que les fonctions esters COOR₁₁ ou COOR₁₂ soient hydrolysables en fonctions acides et le composé de formule III répond à la formule:

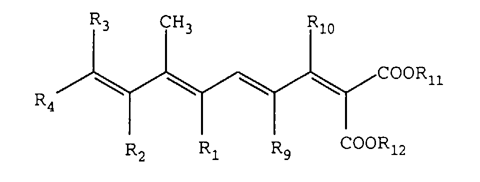

18. Procédé de préparation selon la revendication 17, caractérisé en ce que le composé de formule Il est un ester d'acide isopropylidène malonique.

19. Procédé de préparation selon l'une des revendications 15 à 18, caractérisé en ce que l'agent activant l'hydrogène du méthylène ou du méthyle est choisi parmi les hydroxydes d'ammonium quaternaire, les amidures alcalins.

20. Procédé selon l'une des revendications 15 à 19, caractérisé en ce que les rapports molaires I/II sont compris entre 0,2 et 2.

21. Procédé de préparation de composés choisis parmi les rétinoïdes, les caroténoïdes et leurs analogues structuraux respectifs, caractérisé en ce qu'on hydrolyse le composé de formule III selon la revendication 15, pour obtenir le composé de formule IV 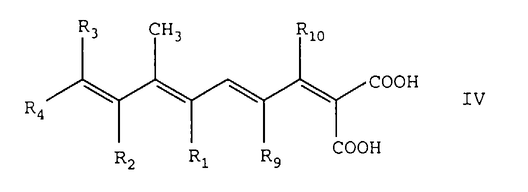 qui est ensuite décarboxylé pour obtenir le composé de formule V 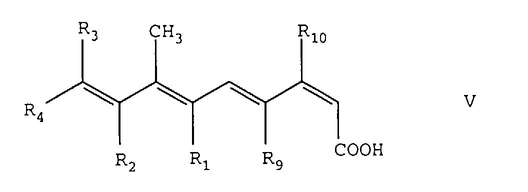 ou VI

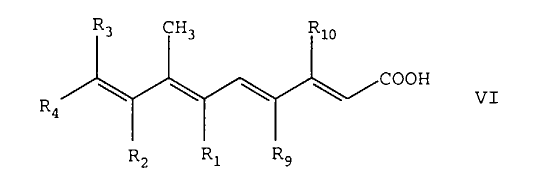

22. Procédé de préparation des composés selon l'une des revendications 1 à 13, caractérisé en ce qu'on fait réagir une cétone éthylénique de formule VII: 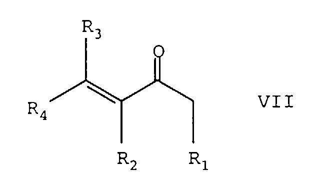 avec un formiate de formule HCOOR₁₃
R₁₃ étant un reste alkyle linéaire ou ramifié en présence d'une base alcaline pour obtenir le composé de formule VIII 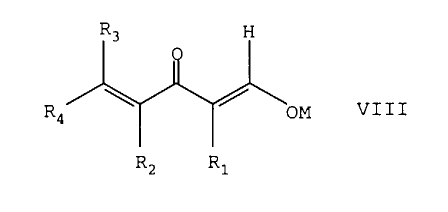 M étant un métal alcalin ou un atome d'hydrogène.
puis acétalisation pour obtenir le composé de formule IX 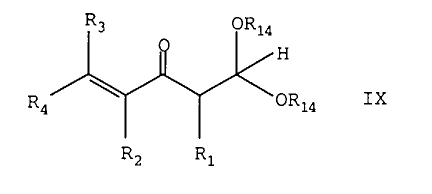 R₁₄ étant des radicaux alkyle linéaires ou ramifiés ou représentant un cycle comme (CH₂)ₙ avec n = 1, 2, 3, 4.
obtention du composé de formule X par une réaction de méthylénation, notamment par une réaction de Wittig avec un halogénure de méthyle triphényl phosphonium ou de méthyle trialkyl-phosphonium. 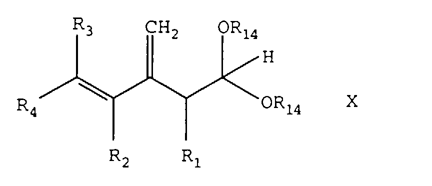 puis hydrolyse pour obtenir le composé de formule I.

23. Composés utiles notamment dans le procédé d'obtention selon la revendication 22, caractérisés en ce qu'ils répondent aux formules VIII, IX ou X, dans laquelle R₁, R₂, R₃, ont l'une des significations des revendications 1 à 10 et R₁₄ et M ont l'une des significations de la revendication 22.
